# EUROPÄISCHE PATENTANMELDUNG

(11) **EP 3 106 203 A1**
(43) Veröffentlichungstag der Anmeldung: **21.12.2016**
(21) Anmeldenummer: 16173296.1
(22) Anmeldetag: 07.06.2016
(51) Int. Cl.: A61N 1/372

(54) **IMPLANTIERBARES MEDIZINISCHES GERÄT UND VERFAHREN ZUM PAAREN EINES IMPLANTIERBAREN MEDIZINISCHEN GERÄTES UND EINES EXTERNEN GERÄTES**

(30) Priorität: 16.06.2015 US 201562180053 P
(71) Anmelder: BIOTRONIK SE & Co. KG, 12359 Berlin (DE)
(72) Erfinder: Gothe, Christian, 12437 Berlin (DE); Fuß, Thomas, 12161 Berlin (DE)
(74) Vertreter: Galander, Marcus

(57) **Zusammenfassung**

Die Erfindung betrifft ein implantierbares medizinisches Gerät (IMD) (100), das eine Steuereinheit (110) aufweist, einen permanenten Speicher (120) für eine Gerätekennung IID, eine Speichereinheit (130) zum Speichern einer Gerätekennung EID eines externen Gerätes, eine Nahfeld-Datenkommunikationseinheit (140) und eine Fernfeld-Datenkommunikationseinheit (150). Die Steuereinheit (110) ist gemeinsam mit der Nahfeld-Datenkommunikationseinheit (140) dazu ausgebildet, ein drittes Gerät im Nahfeld des IMD (100) zu erfassen und im Rahmen einer Nahfeld-Datenkommunikation die Gerätekennung IID des IMD auf das dritte Gerät zu übertragen. Die Steuereinheit (110) ist weiterhin dazu ausgebildet, nach einer Nahfeld-Datenkommunikation mit einem dritten Gerät über die Fernfeld-Datenkommunikationseinheit (150) empfangene Nachrichten daraufhin zu überprüfen, ob diese eine Fernfeld-Start-Nachricht darstellen und eine Gerätekennung IID des IMD enthalten, die der in dem permanenten Speicher (120) gespeicherten Gerätekennung entspricht. In diesem Fall, ist die Steuereinheit ( 110) des IMD (100) dazu ausgebildet, eine Fernfeld-Antwort-Nachricht auszusenden, die die Gerätekennung IID des IMG sowie die Gerätekennung EID des externen Gerätes enthält.

## Beschreibung

Die Erfindung betrifft ein Verfahren zum Paaren eines implantierbaren medizinischen Gerätes mit einem externen Gerät. Außerdem betrifft die Erfindung ein System umfassend ein implantierbares medizinisches Gerät und ein externes Gerät. Weiterhin betrifft die Erfindung ein implantierbares medizinisches Gerät.

Das implantierbare medizinische Gerät kann beispielsweise ein implantierbarer Herzstimulator, wie beispielsweise ein Herzschrittmacher, oder ein implantierbarer Cardioverter/Defibrillator (ICD) sein. Derartige Geräte sind typischerweise als aktive Geräte mit einer elektronischen Steuerung versehen, die Daten erfasst und speichert und/oder programmierbar ist. Um Programmdaten zu einem derartigen implantierbaren medizinischen Gerät zu übertragen oder aus dem implantierbaren medizinischen Gerät erfasste Daten auszulesen, ist typischerweise ein externes Gerät vorgesehen, welches ein Programmiergerät sein kann.

Zum Übertragen von Programmdaten an das implantierbare medizinische Gerät oder zum Übertragen von in dem implantierbaren medizinischen Gerät gespeicherten Daten zu dem externen Gerät wird typischerweise zwischen beiden Geräten eine drahtlose Fernfeld-Datenkommunikation aufgebaut, die unidirektional oder bidirektional sein kann. Damit eine derartige Fernfeld-Datenkommunikation nicht zufällig zwischen zwei Geräten entsteht, zwischen denen zu einem jeweiligen Zeitpunkt keine Fernfeld-Datenkommunikation vorgesehen ist, geht einer jeweiligen Fernfeld-Datenkommunikation ein sogenanntes Pairing voraus, also ein Paaren, bei dem das implantierbare medizinische Gerät und das externe Gerät, die miteinander per Fernfeld-Datenkommunikation kommunizieren sollen, für diese Fernfeld-Datenkommunikation einander zugeordnet werden. Für ein derartiges Pairing ist typischerweise ein Programmierkopf vorgesehen, der mittels eines Kabels mit dem externen Gerät verbunden ist und der dazu dient, zwischen dem jeweiligen implantierbaren medizinischen Gerät und dem Programmierkopf eine Nahfeld-Datenkommunikation herzustellen, bei der das implantierbare medizinische Gerät und das externe Gerät solche Daten miteinander austauschen, die dazu geeignet sind, die beiden Geräte für die jeweilige Fernfeld-Datenkommunikation einander zuzuordnen.

Bei neueren Programmiergeräten kommen auch anstelle der drahtgebundenen Programmierköpfe drahtlose Programmierköpfe zum Einsatz. Hierbei ist ein drahtloser Programmierkopf durch eine drahtlose Anbindung (Kopplung) dauerhaft einem bestimmten Programmiergerät zugeordnet. Beim Einsatz eines drahtlosen Programmierkopfs besteht die Gefahr, dass die Programmierköpfe zweier Programmiergeräte vertauscht werden. Eine Vertauschung der drahtlosen Programmierköpfe wird dann problematisch, wenn zwei Programmiergeräte mit vertauschten drahtlosen Programmierköpfen in unmittelbarer Nähe zueinander und zeitgleich für eine Nachsorge zum Einsatz kommen. Durch die Vertauschung kann in einem solchen Fall beim Pairing versehentlich das Implantat des benachbarten Patienten mit dem eigenen Programmiergerät verbunden werden. Hierdurch wird im ungünstigsten Fall versehentlich das Implantat des benachbarten Patienten programmiert.

Aufgabe der Erfindung ist es, ein alternatives, insbesondere einfaches und sicheres Pairing von implantierbaren medizinischen Gerät und externem Gerät zu ermöglichen.

Erfindungsgemäß wird diese Aufgabe durch ein Verfahren zum Pairing eines implantierbaren medizinischen Gerätes mit einem externen Gerät gelöst, von denen das implantierbare medizinische Gerät für eine drahtlose Fernfeld-Datenkommunikation mit dem externen Gerät ausgerüstet ist und eine eindeutige Gerätekennung IID aufweist, und von denen das externe Gerät für eine drahtlose Fernfeld-Datenkommunikation mit dem implantierbaren medizinischen Gerät ausgerüstet ist und eine eindeutige Gerätekennung EID besitzt. Die eindeutige Gerätekennung IID ist somit die jeweilige eindeutige Gerätekennung des implantierbaren medizinischen Gerätes, während die Gerätekennung EID die jeweilige eindeutige Gerätekennung des externen Gerätes ist.

Das Verfahren umfasst die folgenden Schritte:
- Nahfeld-Übertragung einer der Gerätekennungen, IID oder EID, von dem implantierbaren medizinischen Gerät bzw. dem externen Gerät auf ein drittes Gerät im Nahfeld des implantierbaren medizinischen Gerätes oder des externen Gerätes,
- Zwischenspeichern der jeweiligen Gerätekennung IID oder EID auf dem dritten Gerät,
- Nahfeld-Übertragung der Gerätekennung IID von dem dritten Gerät auf das externe Gerät oder der Gerätekennung EID vom dritten Gerät auf das implantierbare medizinische Gerät und
- Initiieren einer Fernfeld-Datenkommunikation zwischen dem externen Gerät mit der Gerätekennung EID und dem implantierbaren medizinischen Gerät mit der Gerätekennung IID.

Mit anderen Worten überträgt eines der Geräte des Systems, nämlich das implantierbare medizinische Gerät oder das externe Gerät, seine eigene eindeutige Gerätekennung IID oder EID auf ein drittes Gerät, das sich hierzu im Nahfeld des jeweiligen Gerätes, also des implantierbaren medizinischen Gerätes oder des externen Gerätes, befinden muss. In dem dritten Gerät wird die jeweilige eindeutige Gerätekennung, IID oder EID, zwischengespeichert und das dritte Gerät wird in die Nähe des jeweils anderen Gerätes und damit in dessen Nahfeld gebracht. Das jeweils andere Gerät ist dasjenige Gerät, dessen eindeutige Gerätekennung nicht auf dem dritten Gerät gespeichert ist. Sobald das dritte Gerät in die Nähe des jeweiligen anderen Gerätes gebracht ist, wird die in dem dritten Gerät gespeicherte eindeutige Gerätekennung auf das jeweils andere Gerät übertragen, so dass in diesem jeweils anderen Gerät sowohl dessen jeweilige eigene eindeutige Gerätekennung vorliegt als auch die mittels des dritten Gerätes übertragene eindeutige Gerätekennung des ersten Gerätes.

Wie zu erkennen ist, umfasst das Verfahren zwei Varianten, nämlich zum einen die Variante, bei der das dritte Gerät zunächst in die Nähe und damit das Nahfeld des implantierbaren medizinischen Gerätes gebracht wird und von dem implantierbaren medizinischen Gerät dessen eindeutige Gerätekennung IID per Nahfeld-Datenkommunikation empfängt, zwischenspeichert, und anschließend im Nahfeld des externen Gerätes die eindeutige Gerätekennung IID des implantierbaren medizinischen Gerätes an das externe Gerät via Nahfeld-Datenkommunikation überträgt. In diesem Fall liegen dann in dem externen Gerät sowohl dessen eigene Gerätekennung EID als auch die mittels des dritten Gerätes empfangene Gerätekennung IID des implantierbaren medizinischen Gerätes vor. Das externe Gerät kann daraufhin per Fernfeld-Datenkommunikation eine Fernfeld-Startnachricht an das implantierbare medizinische Gerät senden, wobei diese Fernfeld-Startnachricht sowohl die eindeutige Gerätekennung IID desjenigen implantierbaren medizinischen Gerätes enthält, an das die Fernfeld-Startnachricht gerichtet ist, als auch die eindeutige Gerätekennung EID des aussendenden externen Gerätes.

Bei der anderen Verfahrensvariante wird das dritte Gerät zunächst in das Nahfeld des externen Gerätes gebracht und empfängt per Nahfeld-Datenkommunikation die eindeutige Gerätekennung EID des externen Gerätes. Diese eindeutige Gerätekennung EID wird in dem dritten Gerät zwischengespeichert. Das dritte Gerät wird anschließend in die Nähe und damit in das Nahfeld des implantierbaren medizinischen Gerätes gebracht und überträgt per Nahfeld-Datenkommunikation die eindeutige Gerätekennung EID des externen Gerätes an das implantierbare medizinische Gerät. Anschließend liegen in dem implantierbaren medizinischen Gerät sowohl dessen eigene eindeutige Gerätekennung IID als auch die vom dritten Gerät empfangene eindeutige Gerätekennung EID des externen Gerätes vor. Daraufhin kann das implantierbare medizinische Gerät eine Fernfeld-Start-Nachricht aussenden, die an dasjenige externe Gerät gerichtet ist, dessen eindeutige Gerätekennung EID das implantierbare Gerät von dem dritten Gerät empfangen hat. Diese Fernfeld-Start-Nachricht enthält sowohl die eindeutige Gerätekennung EID desjenigen externen Gerätes, an das sie gerichtet ist, als auch die eindeutige Gerätekennung IID desjenigen implantierbaren medizinischen Gerätes, das die Fernfeld-Start-Nachricht aussendet.

Wie sich aus der vorstehenden Schilderung ergibt, ist es vorteilhaft, wenn das Initiieren der Fernfeld-Datenkommunikation ein Aussenden einer Fernfeld-Start-Nachricht durch dasjenige Gerät umfasst, das von dem dritten Gerät die eindeutige Gerätekennung EID bzw. IID empfangen hat, wobei die Fernfeld-Start-Nachricht jeweils sowohl die empfangene Gerätekennung EID bzw. IID als auch die Gerätekennung IID bzw. EID des die Fernfeld-Start-Nachricht aussenden Gerätes umfasst.

Auch ist es vorteilhaft, wenn die jeweils in dem dritten Gerät zwischengespeicherte eindeutige Gerätekennung IID oder EID von dem dritten Gerät gelöscht wird, nachdem das dritte Gerät diese eindeutige Gerätekennung per Nahfeld-Datenkommunikation an das jeweils andere Gerät übertragen hat. Auf diese Weise wird sichergestellt, dass auf dem dritten Gerät nicht eine eindeutige Gerätekennung gespeichert bleibt, die eine ungewollte Paarung (ein ungewolltes Pairing) von externem Gerät und implantierbarem medizinischen Gerät verursachen könnte.

Auch ist es vorteilhaft, wenn die jeweils in dem dritten Gerät zwischengespeicherte eindeutige Gerätekennung IID oder EID von dem dritten Gerät gelöscht wird, nachdem das dritte Gerät diese eindeutige Gerätekennung per Nahfeld-Datenkommunikation in einer vorgegebenen Zeit nicht an das jeweils andere Gerät übertragen hat. Auf diese Weise wird sichergestellt, dass auf dem dritten Gerät nicht eine eindeutige Gerätekennung gespeichert bleibt, die eine ungewollte Paarung (ein ungewolltes Pairing) von externem Gerät und implantierbarem medizinischen Gerät verursachen könnte.

Auch ist es vorteilhaft, wenn die jeweils in dem dritten Gerät zwischengespeicherte eindeutige Gerätekennung IID oder EID von dem dritten Gerät durch eine Einwirkung des Nutzers auf das dritte Gerät gelöscht werden kann. Eine derartige Einwirkung kann durch die Betätigung einer Schaltvorrichtung (z. B. Taster oder Schalter etc.) am dritten Gerät geschehen, welche eine Löschung der Gerätekennung IID oder EID von dem dritten Gerät bewirkt. Auf diese Weise wird sichergestellt, dass auf dem dritten Gerät nicht eine eindeutige Gerätekennung gespeichert bleibt, die eine ungewollte Paarung (ein ungewolltes Pairing) von externem Gerät und implantierbarem medizinischen Gerät verursachen könnte.

Weiterhin ist es vorteilhaft, wenn das Initiieren einer Fernfeld-Datenkommunikation auch ein Aussenden einer Fernfeld-Antwort-Nachricht durch dasjenige Gerät umfasst, das die Fernfeld-Start-Nachricht empfangen hat und dessen Gerätekennung (IID oder EID) in der Fernfeld-Start-Nachricht enthalten ist. Die Fernfeld-Antwort-Nachricht enthält dabei ebenfalls die beiden in der Fernfeld-Start-Nachricht enthaltenen Gerätekennungen, also die Gerätekennungen derjenigen beiden Geräte, also eines implantierbaren medizinischen Gerätes und eines externen Gerätes, die mittels Pairing für eine Fernfeld-Datenkommunikation gepaart werden sollen.

Bei einer bevorzugten Weiterentwicklung des Verfahrens wird in einem ersten Schritt das dritte Gerät zunächst in die Nähe und damit in das Nahfeld des externen Gerätes gebracht, um von dem externen Gerät dessen eindeutige Gerätekennung EID per Nahfeld-Datenkommunikation zu empfangen und zwischenzuspeichern. In einem zweiten Schritt wird das dritte Gerät in die Nähe und damit in das Nahfeld des implantierbaren medizinischen Gerätes gebracht um von dem implantierbaren medizinischen Gerät dessen eindeutige Gerätekennung IID per Nahfeld-Datenkommunikation zu empfangen und ebenfalls zwischenzuspeichern. Weiterhin im Nahfeld des implantierbaren medizinischen Gerätes wird die im dritten Gerät gespeicherte eindeutige Gerätekennung EID des externen Gerätes von dem dritten Gerät an das implantierbare medizinische Gerät via Nahfeld-Datenkommunikation übermittelt. In einem dritten Schritt wird das dritte Gerät abermals in die Nähe und damit in das Nahfeld des externen Gerätes gebracht wo es die eindeutige Gerätekennung IID des implantierbaren medizinischen Gerätes an das externe Gerät via Nahfeld-Datenkommunikation überträgt. Nach diesen Schritten liegen dann sowohl in dem externen Gerät als auch im implantierbaren medizinischen Gerät die beiden Gerätekennungen IID und EID vor. Das externe Gerät kann daraufhin per Fernfeld-Datenkommunikation eine Fernfeld-Startnachricht an das implantierbare medizinische Gerät senden, wobei diese Fernfeld-Startnachricht sowohl die eindeutige Gerätekennung IID desjenigen implantierbaren medizinischen Gerätes enthält, an das die Fernfeld-Startnachricht gerichtet ist, als auch die eindeutige Gerätekennung EID des aussendenden externen Gerätes. Dasjenige implantierbare medizinische Gerät, an das die Fernfeld-Startnachricht gerichtet ist kann anhand der von ihm im zweiten Schritt gespeicherten EID überprüfen, ob die Fernfeld-Startnachricht dasjenige externe Gerät ausgesendet hat, welches im ersten Schritt für die Paarung vorgesehen wurde.

Bei der anderen Verfahrensvariante einer bevorzugten Weiterentwicklung des Verfahrens wird in einem ersten Schritt das dritte Gerät zunächst in die Nähe und damit in das Nahfeld des implantierbaren medizinischen Gerätes gebracht, um von dem implantierbaren medizinischen Gerät dessen eindeutige Gerätekennung IID per Nahfeld-Datenkommunikation zu empfangen und zwischenzuspeichern. In einem zweiten Schritt wird das dritte Gerät in die Nähe und damit in das Nahfeld des externen Gerätes gebracht, um von dem externen Gerät dessen eindeutige Gerätekennung EID per Nahfeld-Datenkommunikation zu empfangen und ebenfalls zwischenzuspeichern. Weiterhin im Nahfeld des externen Gerätes wird die im dritten Gerät gespeicherte eindeutige Gerätekennung IID des implantierbaren medizinischen Gerätes von dem dritten Gerät an das externe Gerät via Nahfeld-Datenkommunikation übermittelt. In einem dritten Schritt wird das dritte Gerät abermals in die Nähe und damit in das Nahfeld des implantierbaren medizinischen Gerätes gebracht wo es die eindeutige Gerätekennung EID des externen Gerätes an das implantierbare medizinische Gerät via Nahfeld-Datenkommunikation überträgt. Nach diesen Schritten liegen dann sowohl in dem externen Gerät als auch im implantierbaren medizinischen Gerät die beiden Gerätekennungen IID und EID vor. Das implantierbare medizinische Gerät kann daraufhin per Fernfeld-Datenkommunikation eine Fernfeld-Startnachricht an das externe Gerät senden, wobei diese Fernfeld-Startnachricht sowohl die eindeutige Gerätekennung EID desjenigen externen Gerätes enthält, an das die Fernfeld-Startnachricht gerichtet ist, als auch die eindeutige Gerätekennung IID des aussendenden implantierbaren medizinischen Gerätes. Dasjenige externe Gerät an das die Fernfeld-Startnachricht gerichtet ist kann anhand der von ihm im zweiten Schritt gespeicherten IID überprüfen, ob die Fernfeld-Startnachricht dasjenige implantierbare medizinische Gerät ausgesendet hat, welches im ersten Schritt für die Paarung vorgesehen wurde.

Erfindungsgemäß wird die Aufgabe auch durch ein System gelöst, das ein implantierbares medizinisches Gerät, ein externes Gerät und ein drittes Gerät umfasst. Das implantierbare medizinische Gerät weist eine eindeutige Gerätekennung IID auf und ist zur drahtlosen Fernfeld-Datenkommunikation mit dem externen Gerät und zur Nahfeld-Datenkommunikation mit dem dritten Gerät ausgerüstet. Das externe Gerät besitzt eine eindeutige Gerätekennung EID und ist zur drahtlosen Fernfeld-Datenkommunikation mit dem implantierbaren medizinischen Gerät und zur Nahfeld-Datenkommunikation mit dem dritten Gerät ausgerüstet. Das dritte Gerät ist zur Nahfeld-Datenkommunikation mit dem implantierbaren medizinischen Gerät und dem externen Gerät ausgerüstet und ausgebildet, wenigstens eine eindeutige Gerätekennung EID und/oder IID von dem externen Gerät bzw. dem implantierbaren medizinischen Gerät via Nahfeld-Datenkommunikation zu empfangen, zwischenzuspeichern und anschließend an das jeweils andere, implantierbare medizinische Gerät oder externe Gerät per Nahfeld-Datenkommunikation zu übertragen.

Zur Lösung der Aufgabe trägt außerdem ein implantierbares medizinisches Gerät bei, das eine Steuereinheit aufweist, einen permanenten Speicher für eine eindeutige Gerätekennung IID, eine Speichereinheit zum Speichern von Daten und insbesondere einer Gerätekennung EID eines externen Gerätes, eine Nahfeld-Datenkommunikationseinheit und eine Fernfeld-Datenkommunikationseinheit. Die Steuereinheit des implantierbaren medizinischen Gerätes ist in Kombination mit der Nahfeld-Datenkommunikationseinheit dazu ausgebildet, ein drittes Gerät im Nahfeld des implantierbaren medizinischen Gerätes zu erfassen und gegebenenfalls im Rahmen einer Nahfeld-Datenkommunikation mit dem dritten Gerät seine Gerätekennung IID an das dritte Gerät zu übertragen. Die Steuereinheit des implantierbaren medizinischen Gerätes ist weiterhin dazu ausgebildet, im Nachgang einer Nahfeld-Datenkommunikation mit einem dritten Gerät über die Fernfeld-Datenkommunikationseinheit gegebenenfalls empfangene Nachrichten daraufhin zu überprüfen, ob diese eine Fernfeld-Start-Nachricht darstellen. Falls dies der Fall ist, ist die Steuereinheit des implantierbaren medizinischen Gerätes dazu ausgebildet, anhand der in der Fernfeld-Start-Nachricht enthaltenen Gerätekennung IID festzustellen, ob die empfangene Fernfeld-Start-Nachricht für dieses implantierbare medizinische Gerät bestimmt ist, um nur dann die in der Fernfeld-Start-Nachricht enthaltene Gerätekennung EID des externen Gerätes, das die Fernfeld-Start-Nachricht ausgesendet hat in die Speichereinheit des implantierbaren medizinischen Gerätes zu übertragen. Weiterhin ist die Steuereinheit des implantierbaren medizinischen Gerätes dazu ausgebildet, eine Fernfeld-Antwort-Nachricht zu generieren und auszusenden, die die Gerätekennung IID des implantierbaren medizinischen Gerätes sowie die in der Speichereinheit gespeicherte Gerätekennung EID des externen Gerätes enthält.

Ein derartiges implantierbares medizinisches Gerät ist dafür geeignet, in einem Verfahren der vorbeschriebenen Art betrieben zu werden.

Des Weiteren trägt zur Lösung der Aufgabe ein implantierbares medizinisches Gerät bei, das eine Steuereinheit aufweist, einen permanenten Speicher für eine eindeutige Gerätekennung IID, eine Speichereinheit zum Speichern von Daten und insbesondere einer Gerätekennung EID eines externen Gerätes, eine Nahfeld-Datenkommunikationseinheit und eine Fernfeld-Datenkommunikationseinheit. Die Steuereinheit des implantierbaren medizinischen Gerätes ist in Kombination mit der Nahfeld-Datenkommunikationseinheit dazu ausgebildet, ein drittes Gerät im Nahfeld des implantierbaren medizinischen Gerätes zu erfassen und gegebenenfalls im Rahmen einer Nahfeld-Datenkommunikation mit dem dritten Gerät eine Gerätekennung EID eines externen Gerätes zu empfangen und in der Speichereinheit zu speichern. Die Steuereinheit des implantierbaren medizinischen Gerätes ist weiterhin dazu ausgebildet, im Nachgang einer Nahfeld-Datenkommunikation mit einem dritten Gerät über die Fernfeld-Datenkommunikationseinheit gegebenenfalls empfangene Nachrichten daraufhin zu überprüfen, ob diese eine Fernfeld-Start-Nachricht darstellen und eine Gerätekennung EID eines externen Gerätes enthalten, die der in der Speichereinheit gespeicherten Gerätekennung EID entspricht. Falls dies der Fall ist, ist die Steuereinheit des implantierbaren medizinischen Gerätes dazu ausgebildet, eine Fernfeld-Antwort-Nachricht zu generieren und auszusenden, die die Gerätekennung IID des implantierbaren medizinischen Gerätes sowie die in der Speichereinheit gespeicherte Gerätekennung EID enthält.

Ein derartiges implantierbares medizinisches Gerät ist dafür geeignet, in einem weiterentwickelten Verfahren der vorbeschriebenen Art betrieben zu werden.

Gemäß einer vorteilhaften Ausführungsvariante überträgt das dritte Gerät neben einer oder mehrerer jeweiliger Gerätekennungen IID und/oder EID auch Chiffrierschlüssel für eine verschlüsselte Datenkommunikation zwischen dem implantierbaren medizinischen Gerät und dem externen Gerät. Diese Übertragung kann parallel zur Übertragung der Gerätekennungen EID oder IID stattfinden oder in einem zusätzlichen Schritt. Fand eine Übertragung von Chiffrierschlüsseln statt, so erfolgt die Fernfeld-Datenkommunikation zwischen dem implantierbaren medizinischen Gerät und dem externen Gerät nach erfolgreicher Paarung durch verschlüsselte Datenkommunikation. Optional kann auch bereits die Paarung zwischen dem implantierbaren medizinischen Gerät und dem externen Gerät durch verschlüsselte Datenkommunikation erfolgen

Auch ist es vorteilhaft, wenn die jeweils in dem dritten Gerät zwischengespeicherten Chiffrierschlüssel von dem dritten Gerät gelöscht werden, nachdem das dritte Gerät diese Chiffrierschlüssel per Nahfeld-Datenkommunikation an das jeweils andere Gerät übertragen hat. Auf diese Weise wird sichergestellt, dass auf dem dritten Gerät nicht ein Chiffrierschlüssel gespeichert bleibt.

Weiterhin ist es vorteilhaft, wenn die jeweils in dem dritten Gerät zwischengespeicherten Chiffrierschlüssel von dem dritten Gerät nach einer vorgegebenen Zeitspanne automatisch gelöscht werden. Auf diese Weise wird sichergestellt, dass auf dem dritten Gerät nicht ein Chiffrierschlüssel gespeichert bleibt.

Ebenso ist es bevorzugt, wenn das vorbeschriebene Verfahren um weitere Schritte des Paarens erweitert wird, indem ein externes Gerät mittels des dritten Gerätes mit mehreren implantierbaren medizinischen Geräten gepaart wird. Das jeweilige Pairing kann dabei so erfolgen, wie dies zuvor beschrieben ist.

Ein derartiges Verfahren hat den Vorteil, dass mehrere implantierbare medizinische Geräte gleichzeitig Daten mit einem externen Gerät austauschen können. Alternativ oder zusätzlich hat ein solches Verfahren den Vorteil, dass mehrere implantierbare medizinische Geräte gleichzeitig über ein externes Gerät mit einem Service Center verbunden werden können.

Ebenso ist es bevorzugt, wenn das vorbeschriebene Verfahren um weitere Schritte des Paarens erweitert wird, indem ein implantierbares medizinisches Gerät mittels des dritten Gerätes mit mehreren externen Geräten gepaart wird. Das jeweilige Pairing kann dabei so erfolgen, wie dies zuvor beschrieben ist.

Ein derartiges Verfahren hat den Vorteil, dass ein implantierbares medizinisches Gerät mit mehreren externen Geräten verbunden werden kann. Es sind mehrere Situationen denkbar, in denen ein implantierbares medizinisches Gerät mit mehreren externen Geräten verbunden werden soll. Exemplarisch aber nicht erschöpfend seien hier genannt: Eine Verbindung zwischen einem implantierbarem medizinischen Gerät und einem Patientengerät, z. B. ein Kommunikationsgerät zu einem Service Center wird z.B. für eine Langzeitüberwachung des Patienten gewünscht (Home Monitoring). Zusätzlich soll das implantierbare medizinische Gerät mit einem Programmiergerät verbunden werden. Weiterhin kann es wünschenswert sein, für Schulungszwecke ein implantierbares medizinisches Gerät gleichzeitig mit zwei oder mehr Programmiergeräten zu verbinden. Eine Verbindung zwischen einem implantierbarem medizinischen Gerät und mehreren Patientengeräten, z. B. Kommunikationsgeräte zu einem Service Center können den Aufenthaltsbereich erweitern innerhalb dessen ein implantierbares medizinisches Gerät permanent Kontakt zu einem Service Center halten kann.

Weiterhin ist es vorteilhaft, wenn das dritte Gerät dazu ausgebildet ist, einen zeitlich begrenzten, einmaligen Zufallswert zu erzeugen und bei einer jeweiligen Nahfeld-Datenkommunikation an das implantierbare medizinische Gerät und das externe Gerät zu übertragen, so dass der zeitlich begrenzte, einmalige Zufallswert zum Zeitpunkt des Pairings sowohl in dem implantierbaren medizinischen Gerät als auch in dem externen Gerät vorliegt und im Rahmen der per Fernfeld-Datenkommunikation ausgetauschten Fernfeld-Start-Nachricht und/oder Fernfeld-Antwort-Nachricht an das jeweilige andere Gerät übertragen werden kann, so dass eine anschließende Fernfeld-Datenkommunikation nur bei Übereinstimmung der übertragenen, zeitlich begrenzten einmaligen Zufallswerte ausgelöst wird. Sollten die zeitlich begrenzten, einmaligen Zufallswerte nicht übereinstimmen, wird das Pairing des implantierbaren medizinischen Gerätes mit dem externen Gerät abgebrochen.

Weiterhin ist es vorteilhaft, wenn das dritte Gerät dazu ausgebildet ist, bei einer Nahfeld-Datenkommunikation mit dem implantierbaren medizinischen Gerät und mit dem externen Gerät bei dem jeweiligen Gerät vor der Übertragung der IID und/oder EID an das dritte Gerät die Erzeugung einer jeweiligen Zufallszahl durch einen Zufallszahlengenerator im implantierbaren medizinischen Gerät und/oder durch einen Zufallszahlengenerator im externen Gerät zu initiieren, die dann vom implantierbaren medizinischen Gerät als IID und vom externen Gerät als EID genutzt wird und noch in der bestehenden Nahfeld-Datenkommunikation an das dritte Gerät übertragen wird. Die so erzeugten Werte für IID und EID bleiben bis zur nächsten Nahfeld-Datenkommunikation mit dem dritten Gerät jeweils gültig.

Zusätzlich zu den Gerätekennungen IID und EID oder alternativ zu den Gerätekennungen IID und EID kann es vorteilhaft sein, wenn eine Zufallszahl zur Identifikation einer Fernfeld-Datenkommunikation als Session-ID SID genutzt wird. Eine Session-ID SID bleibt bis zur Beendigung der Fernfeld-Datenkommunikation zwischen einem implantierbaren medizinischen Gerät und einem externen Gerät gültig.

Darüber hinaus ist es vorteilhaft, wenn die durch das dritte Gerät getriggerte Neuerzeugung der IID im implantierbaren medizinischen Gerät oder der EID im externen Gerät dann unterdrückt wird, wenn bereits eine Verbindung des implantierbaren medizinischen Geräts zu einem externen Gerät oder eine Verbindung des externen Geräts zu einem implantierbaren medizinischen Gerät besteht und eine weitere Verbindung aufgebaut werden soll.

Alternativ kann es vorteilhaft sein, wenn ein implantierbares medizinisches Gerät gleichzeitig Kommunikationswege zu mehreren externen Geräten unterhält oder wenn ein externes Gerät gleichzeitig Kommunikationswege zu mehreren implantierbaren medizinischen Geräten unterhält, dass für jeden Kommunikationsweg eine eigene IID und/oder EID genutzt wird.

Die Erfindung soll nun anhand eines Ausführungsbeispiels mit Bezug auf die Figuren näher erläutert werden. Diese zeigen in
- Fig. 1:: Einen Überblick über ein System mit einem implantierbaren medizinischen Gerät, einem externen Gerät sowie einem dritten Gerät;
- Fig. 2 bis 9:: Verfahrensschritte zum Paaren des implantierbaren medizinischen Gerätes und des externen Gerätes mittels des dritten Gerätes;
- Fig. 10:: eine schematische Darstellung des implantierbaren medizinischen Gerätes mit einigen Komponenten desselben;
- Fig. 11:: eine schematische Darstellung des externen Gerätes mit einigen Komponenten desselben; und
- Fig. 12:: eine schematische Darstellung des dritten Gerätes und einige von dessen Komponenten.

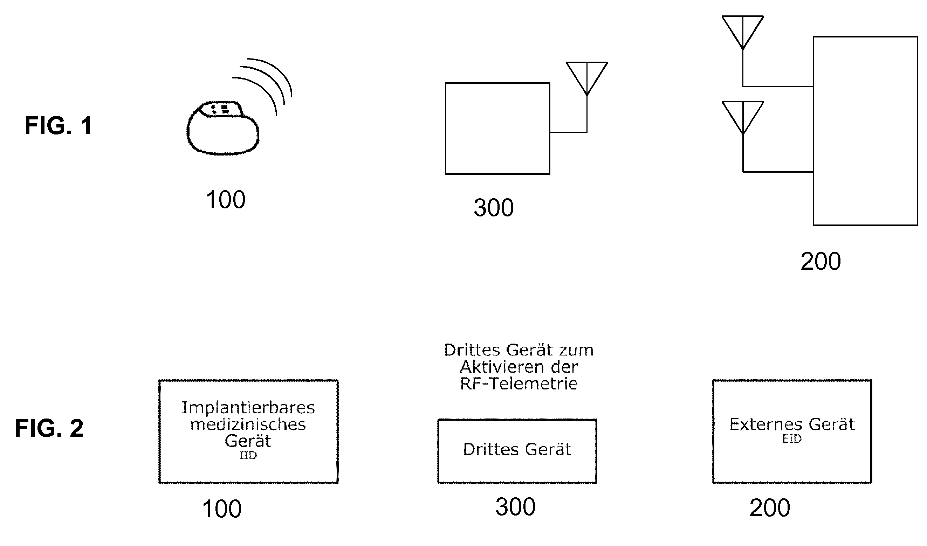

Fig. 1 zeigt ein System mit einem implantierbaren medizinischen Gerät 100, einem externen Gerät 200 und einem dritten Gerät 300. Das implantierbare medizinische Gerät 100 kann beispielsweise ein implantierbarer Herzschrittmacher, ein implantierbarer Neurostimulator, eine implantierbare Medikamentenpumpe oder ein implantierbarer Cardioverter/Defibrillator (ICD) sein. Das externe Gerät 200 kann ein Programmiergerät sein. Das dritte Gerät ist ein Hilfsgerät zum Paaren des implantierbaren medizinischen Geräts 100 mit dem externen Gerät 200.

Das implantierbare Gerät 100 ist so ausgerüstet, dass es per Fernfeld-Datenkommunikation mit dem externen Gerät 200 kommunizieren kann und per Nahfeld-Datenkommunikation mit dem dritten Gerät 300 kommunizieren kann. Das externe Gerät 200 ist so ausgerüstet, dass es mit dem implantierbaren medizinischen Gerät 100 per Fernfeld-Datenkommunikation kommunizieren kann und mit dem dritten Gerät 300 per Nahfeld-Datenkommunikation kommunizieren kann. Das dritte Gerät 300 kann sowohl mit dem externen Gerät 200 als auch mit dem implantierbaren medizinischen Gerät 100 per Nahfeld-Datenkommunikation kommunizieren.

Nahfeld-Datenkommunikation kann eine kurzreichweitige elektromagnetische Datenkommunikation, wie beispielsweise NFC, eine induktive Datenkommunikation wie beispielsweise die von Programmiergeräten bekannte Spulentelemetrie oder auch eine akustische Datenkommunikation im hörbaren oder Ultraschallbereich sein. In jedem Fall ist die Nahfeld-Datenkommunikation eine derartige Datenkommunikation, die es erfordert, dass die beiden miteinander kommunizierenden Geräte sich in unmittelbarer Nähe zueinander befinden.

Fernfeld-Datenkommunikation ist hingegen eine Datenkommunikation beispielsweise via Medical Implant Communication Service (MICS), Bluetooth, Medical Bluetooth, WLAN, ZigBee oder ähnlichem, die eine etwas größere Distanz zwischen den miteinander kommunizierenden Geräten erlaubt. Diese etwas größere Distanz und damit die Reichweite der Fernfeld-Datenkommunikation beträgt beispielsweise wenige Meter, während die Nahfeld-Datenkommunikation eine Reichweite von wenigen Zentimetern bis zu maximal einem Meter hat.

In den in Figuren 2 bis 9 dargestellten Verfahrensschritten ist das externe Gerät 200 als "Programmer" bezeichnet, das implantierbare medizinische Gerät 100 als "Device" und das dritte Gerät 300 als "Magic Key".

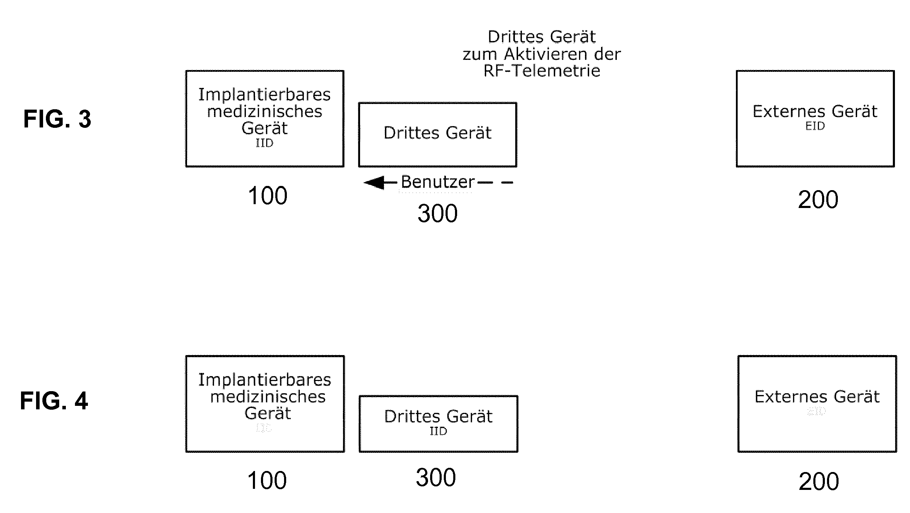

In dem in Figuren 2 bis 9 dargestellten Ausführungsbeispiel eines Verfahrens zum Paaren des implantierbaren medizinischen Gerätes 100 mit dem externen Gerät 200 wird das dritte Gerät 300 zunächst in die Nähe des implantierbaren medizinischen Gerätes 100 gebracht; siehe Fig. 3. Daraufhin erfolgt eine Nahfeld-Datenübertragung zwischen dem implantierbaren medizinischen Gerät 100 und dem dritten Gerät 300, bei der das implantierbare medizinische Gerät 100 seine eigene eindeutige Gerätekennung IID zu dem dritten Gerät 300 überträgt; siehe Fig. 4. Anschließend ist in dem dritten Gerät 300 die eindeutige Gerätekennung IID des implantierbaren medizinischen Geräts 100 gespeichert; siehe Fig. 4.

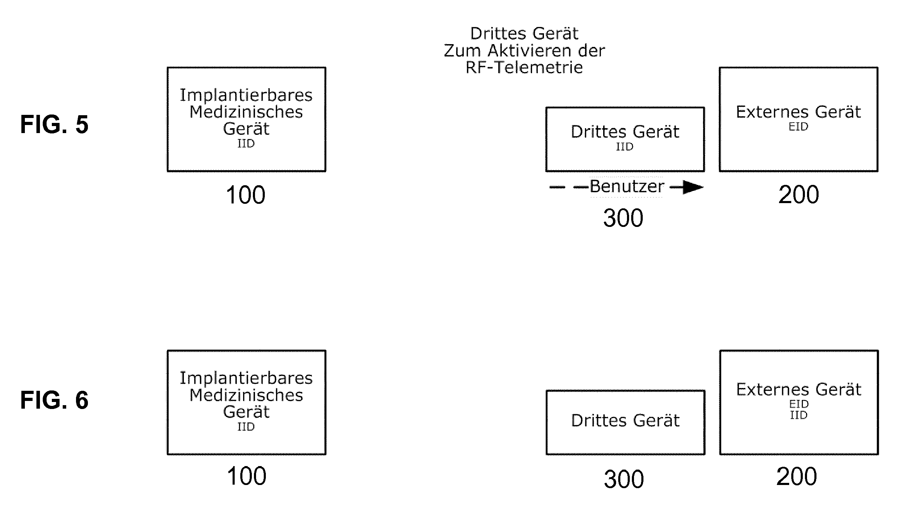

In einem nächsten Schritt wird dann das dritte Gerät 300 in die Nähe des externen Geräts 200 gebracht; siehe Fig. 5.

Daraufhin wird die in dem dritten Gerät 300 zwischengespeicherte eindeutige Gerätekennung IID des implantierbaren medizinischen Gerätes 100 zu dem externen Gerät 200 übertragen. Dies geschieht wiederum auch auf dem Wege der Nahfeld-Datenkommunikation; siehe Fig. 6. Jetzt kann eine Fernfeld-Datenkommunikation beginnen.

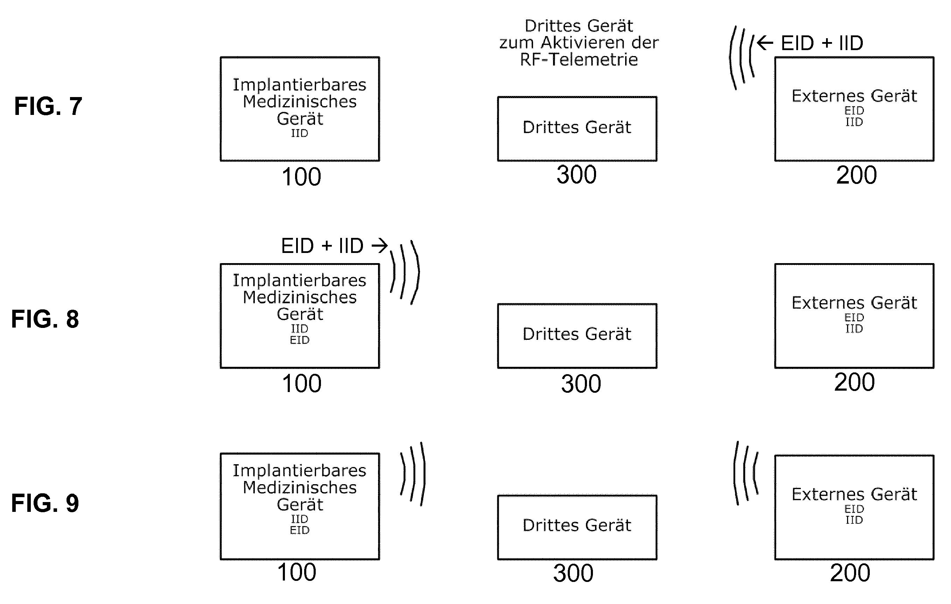

Zum Initiieren der Fernfeld-Datenkommunikation sendet das externe Gerät 200 eine Fernfeld-Start-Nachricht via Fernfeld-Datenkommunikation an das implantierbare medizinische Gerät 100 aus. Diese Fernfeld-Start-Nachricht enthält sowohl die eindeutige Gerätekennung EID des externen Gerätes 200 als auch die eindeutige Gerätekennung IID des implantierbaren medizinischen Gerätes 100; siehe Fig. 7. Das implantierbare medizinische Gerät 100 kann somit überprüfen, ob eine jeweils von ihm empfangene Fernfeld-Start-Nachricht die eigene Gerätekennung IID enthält. Falls dies der Fall ist, generiert und sendet das implantierbare medizinische Gerät 100 eine Fernfeld-Antwort-Nachricht an das externe Gerät 200. Dies geschieht ebenfalls auf dem Wege der Fernfeld-Datenkommunikation. Die Fernfeld-Antwort-Nachricht enthält wiederum die Gerätekennung IID des implantierbaren medizinischen Gerätes sowie die Gerätekennung EID des externen Gerätes 200; siehe Fig. 8.

Diese Fernfeld-Antwort-Nachricht wird von dem externen Gerät 200 empfangen. Das externe Gerät 200 kann daraufhin die mit der Fernfeld-Antwort-Nachricht empfangenen Gerätekennungen EID und IID mit der eigenen Gerätekennung EID sowie mit derjenigen Gerätekennung IID des implantierbaren medizinischen Gerätes 100 vergleichen, die das externe Gerät 200 zuvor via Nahfeld-Datenkommunikation von dem dritten Gerät 300 empfangen hat. Falls die Überprüfung dieser mittels der Fernfeld-Antwort-Nachricht via Fernfeld-Datenkommunikation empfangenen Gerätekennungen IID und EID eine Übereinstimmung mit dem im externen Gerät 200 gespeicherten Gerätekennungen EID und IID ergibt, ist die Paarung erfolgreich abgeschlossen und eine weitere uni- oder bidirektionale Fernfeld-Datenkommunikation zum Austausch von Daten, beispielsweise Programmdaten, zwischen dem externen Gerät 200 und dem implantierbaren medizinischen Gerät 100 kann stattfinden; siehe Fig. 9.

In einem weiteren Ausführungsbeispiel wird dafür gesorgt, dass bereits vor der Initialisierung der Fernfeld-Datenkommunikation sowohl dem implantierbaren medizinischen Gerät 100 als auch dem externen Gerät 200 beide Gerätekennungen IID und EID bekannt sind.

In diesem weiteren Ausführungsbeispiel eines Verfahrens zum Paaren des implantierbaren medizinischen Gerätes 100 mit dem externen Gerät 200 wird das dritte Gerät 300 zunächst in die Nähe des externen Geräts 200 gebracht. Gegebenenfalls kann das dritte Gerät 300 sich auch bereits an dem externen Gerät 200 befinden, beispielsweise ein Stift (Stylus) zum Bedienen des externen Geräts 200 sein. Das dritte Gerät 300 empfängt zunächst eine eindeutige Gerätekennung EID des externen Geräts 200. Dies geschieht auf dem Wege einer Nahfeld-Datenkommunikation zwischen dem externen Gerät 200 und dem dritten Gerät 300. Anschließend wird die eindeutige Gerätekennung EID des externen Geräts 200, in dem dritten Gerät 300 zwischengespeichert.

Das dritte Gerät 300 mit der zwischengespeicherten Gerätekennung EID des externen Gerätes 200 wird daraufhin in die Nähe des implantierbaren medizinischen Gerätes 100 gebracht.

Daraufhin erfolgt eine Nahfeld-Datenübertragung zwischen dem implantierbaren medizinischen Gerät 100 und dem dritten Gerät 300, bei dem das dritte Gerät 300 die eindeutige Gerätekennung EID des externen Geräts 200 zu dem implantierbaren medizinischen Gerät 100 überträgt. Außerdem überträgt das implantierbare medizinische Gerät 100 seine eigene eindeutige Gerätekennung IID via Nahfeld-Datenübertragung zu dem dritten Gerät 300. Anschließend sind in dem dritten Gerät 300 sowohl die eindeutige Gerätekennung EID des externen Geräts 200 als auch die eindeutige Gerätekennung IID des implantierbaren medizinischen Geräts 100 gespeichert.

In einem nächsten Schritt wird dann das dritte Gerät 300 wieder in die Nähe des externen Geräts 200 gebracht.

Daraufhin werden die in dem dritten Gerät 300 zwischengespeicherten Gerätekennungen, nämlich wenigstens die eindeutige Gerätekennung IID des implantierbaren medizinischen Gerätes 100 zu dem externen Gerät 200 übertragen. Dies geschieht auch wiederum auf dem Wege der Nahfeld-Datenkommunikation. Vorzugsweise wird ebenfalls die eindeutige Gerätekennung EID des externen Geräts 200 zu dem externen Gerät 200 übertragen. Das externe Gerät 200 kann daraufhin überprüfen, ob die von dem dritten Gerät 300 per Nahfeld-Datenkommunikation auf das externe Gerät 200 übertragene Gerätekennung EID tatsächlich der Gerätekennung EID des jeweiligen externen Geräts 200 entspricht. Falls dies der Fall ist, ist die Zuordnung von externem Gerät 200 und implantierbarem medizinischen Gerät 100 eindeutig und eine Fernfeld-Datenkommunikation kann beginnen.

Zum Initiieren der Fernfeld-Datenkommunikation sendet das externe Gerät 200 eine Fernfeld-Start-Nachricht via Fernfeld-Datenkommunikation an das implantierbare medizinische Gerät 100 aus. Diese Fernfeld-Start-Nachricht enthält wiederum sowohl die eindeutige Gerätekennung EID des externen Gerätes 200 als auch die eindeutige Gerätekennung IID des implantierbaren medizinischen Gerätes 100. Das implantierbare medizinische Gerät 100 kann somit überprüfen, ob eine jeweils von ihm empfangene Fernfeld-Start-Nachricht die eigene Gerätekennung IID und die Gerätekennung EID des externen Gerätes 200 enthält, die zuvor via Nahfeld-Datenkommunikation von dem dritten Gerät 300 auf das implantierbare medizinische Gerät 100 übertragen wurde. Falls dies der Fall ist, generiert und sendet das implantierbare medizinische Gerät 100 eine Fernfeld-Antwort-Nachricht an das externe Gerät 200. Dies geschieht ebenfalls auf dem Wege der Fernfeld-Datenkommunikation. Die Fernfeld-Antwort-Nachricht enthält wiederum die Gerätekennung IID des implantierbaren medizinischen Gerätes 100 sowie die Gerätekennung EID des externen Gerätes 200.

Diese Fernfeld-Antwort-Nachricht wird von dem externen Gerät 200 empfangen. Das externe Gerät 200 kann daraufhin die mit der Fernfeld-Antwort-Nachricht empfangenen Gerätekennungen EID und IID mit der eigenen Gerätekennung EID des externen Gerätes 200 sowie mit derjenigen Gerätekennung IID des implantierbaren medizinischen Gerätes 100 vergleichen, die das externe Gerät 200 zuvor via Nahfeld-Datenkommunikation von dem dritten Gerät 300 empfangen hat. Falls die Überprüfung dieser mittels der Fernfeld-Antwort-Nachricht via Fernfeld-Datenkommunikation empfangenen Gerätekennungen IID und EID eine Übereinstimmung mit dem im externen Gerät 200 gespeicherten Gerätekennungen EID und IID ergibt, ist die Paarung erfolgreich abgeschlossen und eine weitere uni- oder bidirektionale Fernfeld-Datenkommunikation zum Austausch von Daten, beispielsweise Programmdaten, zwischen dem externen Gerät 200 und dem implantierbaren medizinischen Gerät 100 kann stattfinden.

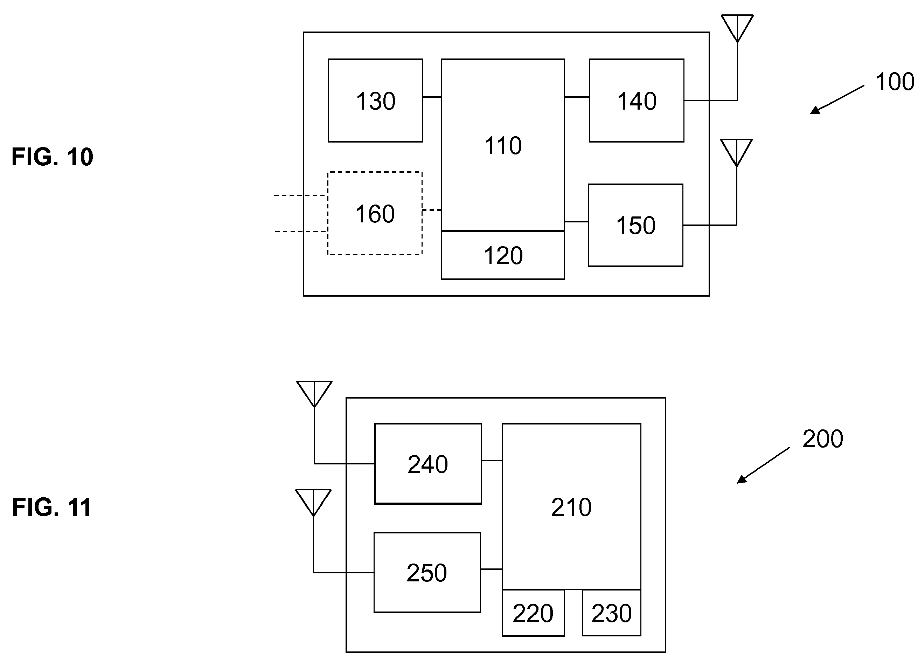

Fig. 10 zeigt in einer schematischen Darstellung einige Komponenten des implantierbaren medizinischen Gerätes 100. Diese Komponenten sind eine Steuereinheit 110 des implantierbaren medizinischen Gerätes, die mit einem permanenten Speicher 120 des implantierbaren medizinischen Gerätes verbunden ist, welche eine eindeutige Gerätekennung IID des implantierbaren medizinischen Gerätes 100 enthält. Außerdem ist die Steuereinheit 110 des implantierbaren medizinischen Gerätes mit einer Speichereinheit 130 des implantierbaren medizinischen Gerätes verbunden, in der Programmdaten oder von dem implantierbaren medizinischen Gerät 100 aufgezeichnete Daten sowie auch via Datenkommunikation empfangene Daten enthalten sein können. Die Speichereinheit 130 des implantierbaren medizinischen Gerätes dient u.a. auch dazu, eine gegebenenfalls empfangene Gerätekennung EID eines externen Gerätes zum Zwecke des Pairings zu speichern.

Außerdem ist die Steuereinheit 110 des implantierbaren medizinischen Gerätes mit einer Nahfeld-Datenkommunikationseinheit 140 des implantierbaren medizinischen Gerätes und einer Fernfeld-Datenkommunikationseinheit 150 des implantierbaren medizinischen Gerätes verbunden. Die Nahfeld-Datenkommunikationseinheit 140 des implantierbaren medizinischen Gerätes dient im hier beschriebenen Ausführungsbeispiel ausschließlich der Nahfeld-Datenkommunikation mit dem dritten Gerät 300, während die Fernfeld-Datenkommunikationseinheit 150 des implantierbaren medizinischen Gerätes dem weiteren Datenaustausch zwischen dem implantierbaren medizinischen Gerät und beispielsweise einem externen Gerät 200 dient.

Nur schematisch angedeutet ist, dass die Steuereinheit 110 des implantierbaren medizinischen Gerätes außerdem noch mit einer oder mehreren Therapie- oder Monitoring-Einheiten 160 des implantierbaren medizinischen Gerätes verbunden sein kann, beispielsweise Stimulations- oder Sensing-Einheiten eines Herzschrittmachers.

Fig. 11 zeigt das externe Gerät 200 mit einigen seiner Komponenten, nämlich einer Steuereinheit 210 des externen Gerätes, die mit einem permanenten Speicher 220 des externen Gerätes verbunden ist, der die erläuterte Gerätekennung EID des externen Gerätes 200 enthält. Weiterhin ist die Steuereinheit 210 des externen Gerätes mit einer Speichereinheit 230 des externen Gerätes verbunden, in der Programmdaten sowie auch via Datenkommunikation empfangene Daten (z. B. von dem implantierbaren medizinischen Gerät 100 aufgezeichnete Daten) enthalten sein können. Die Speichereinheit 230 des externen Gerätes dient u.a. auch dazu, eine gegebenenfalls empfangene Gerätekennung IID eines implantierbaren medizinischen Gerätes zum Zwecke des Pairings zu speichern. Außerdem ist die Steuereinheit 210 des externen Gerätes mit einer Nahfeld-Datenkommunikationseinheit 240 des externen Gerätes sowie mit einer Fernfeld-Datenkommunikationseinheit 250 des externen Gerätes verbunden. Auch hier dient die Nahfeld-Datenkommunikationseinheit 240 des externen Gerätes ausschließlich zur Nahfeld-Datenkommunikation mit dem dritten Gerät 300, während die Fernfeld-Datenkommunikationseinheit 250 des externen Gerätes zur übrigen Datenkommunikation dient, beispielsweise zum Übertragen von Programmierdaten von dem externen Gerät 200 auf das implantierbare medizinische Gerät 100 oder zum Empfangen von dem implantierbaren medizinischen Gerät 100 aufgenommener Daten.

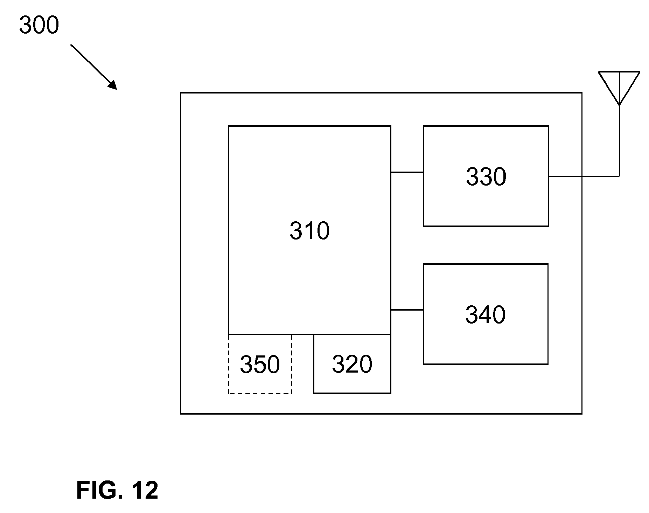

Fig. 12 zeigt schließlich das dritte Gerät 300 und einige seiner Komponenten. Diese sind eine Steuereinheit 310 des dritten Gerätes und eine mit dieser verbundenen Speichereinheit 320 des dritten Gerätes. Die Speichereinheit 320 des dritten Gerätes dient zum temporären Speichern per Nahfeld-Datenkommunikation empfangener Gerätekennungen IID und EID des implantierbaren medizinischen Gerätes 100 bzw. des externen Gerätes 200, der zu übertragenden Chiffrierschlüssel und der aktuell gültigen Zufallszahl.

Außerdem ist die Steuereinheit 310 des dritten Gerätes mit einer Nahfeld-Datenkommunikationseinheit 330 des dritten Gerätes verbunden, über die eine Nahfeld-Datenkommunikation sowohl mit dem externen Gerät 200 als auch mit dem implantierbaren medizinischen Gerät 100 möglich ist, je nachdem in wessen unmittelbarer Nähe sich das dritte Gerät 300 jeweils befindet.

Abhängig von der eingesetzten Technologie kann das dritte Gerät 300 aktiv oder passiv betrieben werden. Das hierfür notwendige Energiemanagement und die erforderlichen Energiespeichereinrichtungen wie Kondensatoren, Batterien oder Akkumulatoren sind aus Gründen der Übersichtlichkeit nicht in Fig. 12 enthalten.

Das dritte Gerät 300 kann außerdem einen Zufallszahlengenerator 340 aufweisen, der einen zeitlich begrenzten, einmaligen Zufallswert erzeugt. Das dritte Gerät 300 ist dann dazu ausgebildet, den zeitlich begrenzten, einmaligen Zufallswert bei einer jeweiligen Nahfeld-Datenkommunikation an das implantierbare medizinische Gerät 100 und das externe Gerät 200 zu übertragen.

Im Rahmen der Fernfeld-Start-Nachricht und/oder der Fernfeld-Antwort-Nachricht wird bei einer weiterentwickelten Variante des Systems und des Verfahrens der jeweilige Zufallswert mitübertragen und nur bei Übereinstimmung dieses Wertes auf beiden Geräten wird die Fernfeld-Datenkommunikation fortgesetzt.

Das dritte Gerät 300 kann weiterhin optional über einen mit der Steuereinheit 310 verbundenen Zeitgeber 350 verfügen. Der Zeitgeber 350 des dritten Gerätes wird von der Steuereinheit 310 genutzt um von dem dritten Gerät 300 in der Speichereinheit 320 des dritten Gerätes gespeicherte Daten nach einer vorgegebenen oder vorgebbaren Zeit zu entfernen. Solche Daten können z. B. sein: Zufallszahlen, die Gerätekennung EID des externen Geräts, die Gerätekennung IID des implantierbaren medizinischen Gerätes, Chiffrierschlüssel oder andere Informationen die auf dem dritten Gerät zum Zwecke des Pairings kurzfristig gespeichert werden.

Weiterhin kann das dritte Gerät 300 über eine Schaltvorrichtung (nicht dargestellt) wie z. B. einen Schalter oder einen Taster verfügen, der mit der Steuereinheit 310 verbunden ist. Die Betätigung dieser Schaltvorrichtung dient dem Zweck, im dritten Gerät gespeicherte Daten (z. B. Zufallszahlen, die Gerätekennung EID des externen Geräts, die Gerätekennung IID des implantierbaren medizinischen Gerätes, Chiffrierschlüssel oder andere Informationen die auf dem dritten Gerät zum Zwecke des Pairings kurzfristig gespeichert werden) aus der Speichereinheit 320 des dritten Gerätes zu entfernen.

Die erfindungsgemäße Lösung bietet folgende Vorteile:
Das dritte Gerät 300 ist
   - kabellos
   - klein
   - leicht
   - desinfizierbar
Das Verfahren erlaubt eine
   - intuitive Bedienung ähnlich der bekannten Lösung und ist
   - günstiger als die Verwendung eines Programmierkopfes.

### Bezugszeichenangabe

- 100: Implantierbares medizinisches Gerät
- 110: Steuereinheit des implantierbaren medizinischen Gerätes
- 120: Permanenter Speicher des implantierbaren medizinischen Gerätes
- 130: Speichereinheit des implantierbaren medizinischen Gerätes
- 140: Nahfeld-Datenkommunikationseinheit des implantierbaren medizinischen Gerätes
- 150: Fernfeld-Datenkommunikationseinheit des implantierbaren medizinischen Gerätes
- 160: Therapie- oder Monitoring-Einheiten des implantierbaren medizinischen Gerätes
- 200: Externes Gerät
- 210: Steuereinheit des externen Gerätes
- 220: Permanenter Speicher des externen Gerätes
- 230: Speichereinheit des externen Gerätes
- 240: Nahfeld-Datenkommunikationseinheit des externen Gerätes
- 250: Fernfeld-Datenkommunikationseinheit des externen Gerätes
- 300: Drittes Gerät
- 310: Steuereinheit des dritten Gerätes
- 320: Speichereinheit des dritten Gerätes
- 330: Nahfeld-Datenkommunikationseinheit des dritten Gerätes
- 340: Zufallszahlengenerator des dritten Gerätes
- 350: Zeitgeber des dritten Gerätes

## Patentansprüche

1. Verfahren zum Pairing eines implantierbaren medizinischen Gerätes mit einem externen Gerät, von denen das implantierbare medizinische Gerät für eine drahtlose Fernfeld-Datenkommunikation mit dem externen Gerät ausgerüstet ist und eine eindeutige Gerätekennung IID aufweist und das externe Gerät für eine drahtlose Fernfeld-Datenkommunikation mit dem implantierbaren medizinischen Gerät ausgerüstet ist und eine eindeutige Gerätekennung EID aufweist, **gekennzeichnet durch** die Verfahrensschritte:
Nahfeldübertragung einer der Gerätekennungen IID oder EID von dem implantierbaren medizinischen Gerät bzw. dem externen Gerät auf ein drittes Gerät im Nahfeld des implantierbaren medizinischen Gerätes oder des externen Gerätes,
Zwischenspeichern der jeweiligen Gerätekennung IID oder EID auf dem dritten Gerät,
Nahfeldübertragung der Gerätekennung IID von dem dritten Gerät auf das externe Gerät oder der Gerätekennung EID von dem dritten Gerät auf das implantierbare medizinische Gerät
und
Initiieren einer Fernfeld-Datenkommunikation zwischen dem externen Gerät mit der Gerätekennung EID und dem implantierbaren medizinischen Gerät mit der Gerätekennung IID.

2. Verfahren nach Anspruch 1, **dadurch gekennzeichnet, dass** eine Gerätekennung IID oder EID von dem dritten Gerät gelöscht wird, nachdem sie von dem dritten Gerät auf das externe Gerät bzw. das implantierbare medizinische Gerät übertragen wurde.

3. Verfahren nach Anspruch 1 oder 2, **dadurch gekennzeichnet, dass** das Initiieren einer Fernfeld-Datenkommunikation ein Aussenden einer Fernfeld-Start-Nachricht durch dasjenige Gerät umfasst, das von dem dritten Gerät die eindeutige Gerätekennung EID bzw. IID empfangen hat, wobei die Fernfeld-Start-Nachricht sowohl die empfangene Gerätekennung EID bzw. IID enthält als auch die Gerätekennung IID bzw. EID des die Fernfeld-Start-Nachricht aussendenden Gerätes, nämlich des implantierbaren medizinischen Gerätes oder des externen Gerätes.

4. Verfahren nach Anspruch 3, **dadurch gekennzeichnet, dass** das Initiieren einer Fernfeld-Datenkommunikation ein Aussenden einer Fernfeld-Antwort-Nachricht durch dasjenige Gerät umfasst, das die Fernfeld-Start-Nachricht empfangen hat und dessen Gerätekennung IID oder EID in der Fernfeld-Start-Nachricht enthalten ist, wobei die Fernfeld-Antwort-Nachricht die beiden in der Fernfeld-Start-Nachricht enthaltenen Gerätekennungen IID und EID enthält.

5. Verfahren nach wenigstens einem der Ansprüche 1 bis 4, **dadurch gekennzeichnet, dass** das dritte Gerät neben einer oder mehrerer jeweiliger Gerätekennungen IID und/oder EID auch einen Chiffrierschlüssel überträgt und die Fernfeld-Datenkommunikation zwischen dem implantierbaren medizinischen Gerät und dem externen Gerät nach erfolgreicher Paarung eine verschlüsselte Datenkommunikation ist.

6. Verfahren nach wenigstens einem der Ansprüche 1 bis 5, **dadurch gekennzeichnet, dass** das Verfahren weitere Schritte des Paarens umfasst, in denen ein jeweiliges externes Gerät mittels des dritten Gerätes mit mehreren implantierbaren medizinischen Geräten gepaart wird.

7. Verfahren nach wenigstens einem der Ansprüche 1 bis 6, **dadurch gekennzeichnet, dass** das dritte Gerät einen zeitlich begrenzten, einmaligen Zufallswert erzeugt und bei einer jeweiligen Nahfeld-Datenkommunikation an das implantierbare medizinische Gerät und das externe Gerät überträgt, so dass der zeitlich begrenzte, einmalige Zufallswert zum Zeitpunkt des Pairings sowohl in dem implantierbaren medizinischen Gerät als auch in dem externen Gerät vorliegt und im Rahmen der per Fernfeld-Datenkommunikation ausgetauschten Fernfeld-Start-Nachricht und/oder Fernfeld-Antwort-Nachricht an das jeweilige andere Gerät übertragen wird, wobei eine anschließende Fernfeld-Datenkommunikation nur bei Übereinstimmung der übertragenen, zeitlich begrenzten einmaligen Zufallswerte ausgelöst wird.

8. Verfahren nach wenigstens einem der Ansprüche 1 bis 7, **dadurch gekennzeichnet, dass** das dritte Gerät zunächst von dem externen Gerät zu dem implantierbaren medizinischen Gerät bewegt wird und dabei die Gerätekennung EID des externen Gerätes zwischengespeichert hat, anschließend die Gerätekennung EID des externen Gerätes an das implantierbare medizinische Gerät überträgt und von dem implantierbaren medizinischen Gerät die Gerätekennung IID empfängt und zwischenspeichert, anschließend wieder in die Nähe des externen Gerätes gebracht wird und an dieses die Gerätekennung IID des implantierbaren medizinischen Gerätes überträgt.

9. Implantierbares medizinisches Gerät (100) mit einer Steuereinheit (110), einem permanenten Speicher (120) für eine eindeutige Gerätekennung IID, einer Speichereinheit (130) zum Speichern einer Gerätekennung EID eines externen Gerätes (200), einer Nahfeld-Datenkommunikationseinheit (140) und einer Fernfeld-Datenkommunikationseinheit (150), **dadurch gekennzeichnet, dass** die Steuereinheit (110) in Kombination mit der Nahfeld-Datenkommunikationseinheit (140) dazu ausgebildet ist, ein drittes Gerät (300) im Nahfeld des implantierbaren medizinischen Gerätes (100) zu erfassen und gegebenenfalls im Rahmen einer Nahfeld-Datenkommunikation mit dem dritten Gerät (300) seine Gerätekennung IID an das dritte Gerät (300) zu übertragen und wobei die Steuereinheit (110) weiterhin dazu ausgebildet ist, im Nachgang einer Nahfeld-Datenkommunikation mit einem dritten Gerät (300) über die Fernfeld-Datenkommunikationseinheit (150) gegebenenfalls empfangene Nachrichten daraufhin zu überprüfen, ob diese eine Fernfeld-Start-Nachricht darstellen und, falls dies der Fall ist, ist die Steuereinheit des implantierbaren medizinischen Gerätes (100) dazu ausgebildet, anhand der in der Fernfeld-Start-Nachricht enthaltenen Gerätekennung IID festzustellen, ob die empfangene Fernfeld-Start-Nachricht für dieses implantierbare medizinische Gerät (100) bestimmt ist, um nur dann die in der Fernfeld-Start-Nachricht enthaltene Gerätekennung EID des externen Gerätes (200), das die Fernfeld-Start-Nachricht ausgesendet hat in die Speichereinheit (130) des implantierbaren medizinischen Gerätes (100) zu übertragen und eine Fernfeld-Antwort-Nachricht zu generieren und auszusenden, die die Gerätekennung IID des implantierbaren medizinischen Gerätes (100) sowie die in der Speichereinheit (130) gespeicherte Gerätekennung EID enthält.

10. Implantierbares medizinisches Gerät (100) gemäß Anspruch 9, **dadurch gekennzeichnet, dass** die Steuereinheit (110) in Kombination mit der Nahfeld-Datenkommunikationseinheit (140) dazu ausgebildet ist, nach der Erfassung eines dritten Gerätes (300) im Nahfeld des implantierbaren medizinischen Gerätes (100) gegebenenfalls im Rahmen einer Nahfeld-Datenkommunikation mit dem dritten Gerät eine Gerätekennung EID eines externen Gerätes zu empfangen und in der Speichereinheit (130) zu speichern, wobei die Steuereinheit (110) weiterhin dazu ausgebildet ist, im Nachgang einer Nahfeld-Datenkommunikation mit einem dritten Gerät über die Fernfeld-Datenkommunikationseinheit (150) gegebenenfalls empfangene Nachrichten daraufhin zu überprüfen, ob diese eine Fernfeld-Start-Nachricht darstellen und eine Gerätekennung EID eines externen Gerätes enthalten, die der in der Speichereinheit (130) gespeicherten Gerätekennung EID entspricht und, falls dies der Fall ist, eine Fernfeld-Antwort-Nachricht zu generieren und auszusenden, die die Gerätekennung IID des implantierbaren medizinischen Gerätes (100) sowie die in der Speichereinheit (130) gespeicherte Gerätekennung EID enthält.

11. System umfassend ein implantierbares medizinisches Gerät (100), ein externes Gerät (200) und ein drittes Gerät (300), von denen
das implantierbare medizinische Gerät (100) eine eindeutige Gerätekennung IID aufweist und zur drahtlosen Fernfeld-Datenkommunikation mit dem externen Gerät (200) und zur Nahfeld-Datenkommunikation mit dem dritten Gerät (300) ausgerüstet ist,
das externe Gerät (200) eine eindeutige Gerätekennung EID aufweist und zur drahtlosen Fernfeld-Datenkommunikation mit dem implantierbaren medizinischen Gerät (100) und zur Nahfeld-Datenkommunikation mit dem dritten Gerät (300) ausgerüstet ist, und
das dritte Gerät (300) zur Nahfeld-Datenkommunikation mit dem implantierbaren medizinischen Gerät (100) und dem externen Gerät (200) ausgerüstet ist und ausgebildet ist, wenigstens eine eindeutige Gerätekennung EID und/oder IID von dem externen Gerät (300) bzw. dem implantierbaren medizinischen Gerät (100) via Nahfeld-Datenkommunikation zu empfangen, zwischenzuspeichern und anschließend an das jeweils andere implantierbare medizinische oder externe Gerät, per Nahfeld-Datenkommunikation zu übertragen.

12. System gemäß Anspruch 11, **dadurch gekennzeichnet, dass** das dritte Gerät (300) dazu ausgebildet ist, bei einer Nahfeld-Datenkommunikation mit dem implantierbaren medizinischen Gerät (100) und mit dem externen Gerät (200) bei dem jeweiligen Gerät vor der Übertragung der IID und/oder EID an das dritte Gerät die Erzeugung einer jeweiligen Zufallszahl durch einen Zufallszahlengenerator im implantierbaren medizinischen Gerät und/oder durch einen Zufallszahlengenerator im externen Gerät zu initiieren, die dann vom implantierbaren medizinischen Gerät als IID und vom externen Gerät als EID genutzt wird und noch in der bestehenden Nahfeld-Datenkommunikation an das dritte Gerät übertragen wird und wobei IID und EID bis zur nächsten Nahfeld-Datenkommunikation mit dem dritten Gerät jeweils gültig bleibt.

13. System gemäß Anspruch 11, **dadurch gekennzeichnet, dass** das dritte Gerät (300) einen Zufallszahlengenerator (340) aufweist, der einen zeitlich begrenzten, einmaligen Zufallswert erzeugt, wobei das dritte Gerät (300) dazu ausgebildet ist, den zeitlich begrenzten, einmaligen Zufallswert bei einer jeweiligen Nahfeld-Datenkommunikation an das implantierbare medizinische Gerät (100) und das externe Gerät (200) zu übertragen.

14. System gemäß einem der Ansprüche 11 bis 13, **dadurch gekennzeichnet, dass** das dritte Gerät (300) ein Bedienstift für das externe Gerät (200) ist.

15. System nach wenigstens einem der Ansprüche 11 bis 14, **dadurch gekennzeichnet, dass** das dritte Gerät (300) neben einer oder mehrerer jeweiliger Gerätekennungen IID und/oder EID auch einen Chiffrierschlüssel überträgt und die Fernfeld-Datenkommunikation zwischen dem implantierbaren medizinischen Gerät (100) und dem externen Gerät (200) nach erfolgreicher Paarung eine verschlüsselte Datenkommunikation ist.
